(19) Europäisches Patentamt
European Patent Office
Office européen des brevets

(11) Publication number: **0 123 352 A1**

# (12) EUROPEAN PATENT APPLICATION

(21) Application number: 84200523.3

(22) Date of filing: 12.04.84

(51) Int. Cl.³: C 07 C 119/042
C 07 C 125/06, C 07 D 251/34
C 08 G 18/73

(30) Priority: 15.04.83 NL 8301341

(43) Date of publication of application:
31.10.84 Bulletin 84/44

(84) Designated Contracting States:
AT BE CH DE FR GB IT LI NL SE

(71) Applicant: STAMICARBON B.V.
Postbus 10
NL-6160 MC Geleen(NL)

(72) Inventor: Ramakers, Johannes A. H. M.
Gozewijnstraat 17
NL-6191 WV Beek (L.)(NL)

(72) Inventor: Tummers, Daniel M. J.
Boerhaavestraat 17
NL-6164 GP Geleen(NL)

(72) Inventor: Vincent, Joseph A. J. M.
Hubertusstraat 73
NL-6191 PB Beek (L.)(NL)

(74) Representative: Leherte, Georges Maurice Lucien Marie et al,
Octrooibureau DSM P.O.Box 9
NL-6160 MA Geleen(NL)

## (54) Aliphatic diisocyanate.

(57) The invention relates to a new diisocyanate, having the formula

$$\underset{\displaystyle CH_3-CH-CH_2-CH_2-CH_2-N=C=O}{\overset{N=C=C}{|}}$$

to the application of this diisocyanate in the preparation of various intermediate products and to the application of this diisocyanate or intermediate products thereof in the preparation of polyurethanes.

EP 0 123 352 A1

ALIPHATIC DIISOCYANATE

The invention relates to a new diisocyanate, to the application of this diisocyanate in the preparation of various intermediate products and to the application of this diisocyanate or intermediate products thereof in the preparation of polyurethanes.

Multifunctional isocyanates often find application in the preparation of polyurethanes, for instance polyurethane foam, elastomers and lacquers.

The isocyanates having no isocyanate group bound to an aromatic nucleus, such as hexane (1,6)diisocyanate, isophorone diisocyanate and trimethyl hexane(1,6)diisocyanate, are applied mainly in high-quality polyurethane products. Examples are lacquers and paints. This type of lacquer can be used for airplanes, car reparation and wood treatment.

Because of the toxicity, volatility and reactivity of the various diisocyanates, it is mostly preferred to use not the diisocyanate itself but rather a reaction product having better properties in this respect. Examples of such reaction products are the adducts of the diisocyanate with a polyol (for instance a diol or a triol), the dimer and the trimer of the diisocyanate, which trimer can advantageously have an isocyanurate structure. In addition, the so-called biuret, a product of the reaction of some diisocyanate molecules with water, can be applied.

In Dutch patent application laid open to public inspection No. 7904202 a process for the preparation of aliphatic diisocyanates is described. The preparation of reaction products of the diisocyanates described in said application presents problems because proportionally many interfering side reactions take place. The same problem occurs when using the diisocyanates described in Dutch patent application laid open to public inspection No. 7906219.

US patent specification No. 3,311,654 discloses various known classes of aliphatic diisocyanates and methods for the preparation thereof. Aliphatic diisocyanates with only primary isocyanate groups are described in German patent application laid open to public inspection No. 2820243.

The disadvantage of the diisocyanates disclosed in the above-

mentioned publications is that no high degree of selectivity can be achieved when reacting them with a compound containing an active hydrogen atom such as a polyol, or water. Numerous side reactions occur, resulting in the loss of at least part of the isocyanate's functionality.

One of the best known aliphatic diisocyanates, 1,6-hexane diisocyanate, can be oligomerized. In doing so, however, in general only relatively low degrees of conversion from the diisocyanate into the desired oligomer can be attained. This makes it necessary to remove the non-converted diisocyanate by distillation, which costs much time and is unfavourable from an energy point of view. Direct oligomerization of the diisocyanate at a high degree of conversion is impossible, as this leads to extensive gel formation.

An alternative to the oligomerization of 1,6-hexane diisocyanate is its reaction with water. This gives rise to biuret formation. Such a reaction being an equilibrium reaction, the free diisocyanate content of the reaction product will increase after some time. This necessitates taking rather expensive safety and health precautions when processing this product.

The object of the invention therefore is to provide a new diisocyanate which does not give rise to the above problems, i.e. a diisocyanate that can easily and at a high selectivity be converted into oligomers or adducts.

The aliphatic diisocyanate according to the invention is, therefore, characterized by the formula

$$\begin{array}{l} \quad N=C=O \\ \quad | \\ CH_3-CH-CH_2-CH_2-CH_2-N=C=O \end{array}$$

Surprisingly, it has been found that this diisocyanate, namely 1,4-pentane diisocyanate can in a simple manner and at a high selectivity be oligomerized. The free diisocyanate content of the oligomer can without many problems be reduced to less than 0.2 wt. % without gel formation.

The diisocyanate according to the invention has the great advantage that it can be reacted selectively through one of both isocyanate groups, for instance with a polyol to obtain a polyisocyanate. It is also quite possible to react three or more diisocyanate molecules with a conventional triol to obtain a high-molecular triisocyanate.

In the formation of this isocyanate no or proportionally few undesired side reactions take place, in contrast with what happens in the formation of 1,6-hexane diisocyanate or of trimethyl hexane (1,6)diisocyanate, where part of the functionality of the isocyanate is lost because the reaction cannot be made to proceed selectively enough.

The same advantage is obtained in the oligomerization of the diisocyanate according to the invention, optionally together with one or more other diisocyanates. In particular the trimerization to an isocyanurate should be mentioned.

The invention therefore also relates to an adduct with one or more terminal isocyanate groups of the diiscocyanate with a compound containing one or more active hydrogen atoms, such as diols or polyols, diamines or polyamines, and water, as well as to an oligomer, for instance the dimer or the trimer of the diisocyanate.

The diisocyanate can be prepared in the manner known for analogous compounds, for instance from the corresponding diamine compound by treatment with phosgene or known reagents. Examples of preparation methods for such analogous compounds are included in the references discussed in the preamble.

By preference diisocyanates are prepared from the corresponding diamines. These diamines can, in their turn, be prepared from the corresponding ketonitrile or aminonitrile.

Important applications of the diisocyanates discussed here are the preparation of solid and microcellular elastomers.

The main application, however, is the preparation of a coating or a lacquer on polyurethane basis This application is very interesting because polyurethanes on the basis of the aliphatic diisocyanates will yellow at a substantially slower rate than polyurethanes on the basis of a diisocyanate the aromatic nucleus of which is bound to one or more isocyanate groups.

In general, the various applications of diisocyanates are known to one skilled in the trade. In literature many recipes are described. Some examples of these are included in European patent applications laid open to public inspection Nos. 4115 en 4116.

<u>Example</u>

205 grams of 1,4-diaminopentane were dissolved in 1600 ml chlorobenzene.

$CO_2$ gas was passed through the solution at room temperature until no $CO_2$ was absorbed anymore. The reaction being exothemic the mixture was cooled. The mixture was subsequently cooled to 0 °C, and 570 grams of phosgene were introduced. The mixture was then heated to room temperature and subsequently 968 grams of N-N-dimethylaniline dissolved in 400 ml of chlorobenzene were added over a period of 1 hour.
After this addition the reaction mixture was heated.
At about 45 ° the reaction mixture spontaneously heats to about 81 °C. The solid phase dissolves with formation of a gas. The mixture is then reacted for another half hour at 100 °C. The mixture is then cooled to 20 °C and extracted twice with 1000 ml of ice water. The organic phase is dried over $Na_2SO_4$. The obtained product was distilled. The fraction distilling between 107 °C and 111 °C at 12-18 mmHg contained 215 gram of 1,4 pentane diisocyanate.

CLAIMS

1. Aliphatic diisocyanate characterized by the formula

N=C=O
|
$CH_3 - CH - CH_2 - CH_2 - CH_2 - N=C=O$

2. Process for the preparation of an aliphatic diisocyanate, characterized in that a diamine of the formula

$NH_2$
|
$CH_3 - CH - CH_2-CH_2-CH_2 - NH_2$

is converted into the corresponding diisocyanate with the aid of phosgene.

3. Oligomer of an aliphatic diisocyanate according to claim 1, optionally in combination with another diisocyanate.

4. Product of the reaction of an aliphatic diisocyanate according to claim 1, with one or more compounds with a reactive H atom.

5. Application of a diisocyanate according to claim 1, in the preparation of a polyurethane.

6. Application of an oligomer according to claim 3, in the preparation of a polyurethane.

7. Application of a reaction product according to claim 4, in the preparation of a polyurethane.

European Patent Office

# EUROPEAN SEARCH REPORT

0123352

Application number

EP 84 20 0523

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y,D | US-A-3 311 654 (A. SAYIGH) * Claims 1,2,5,6 * --- | 1-8 | C 07 C 119/042<br>C 07 C 125/06<br>C 07 D 251/34<br>C 08 G 18/73 |
| Y | DE-A-1 569 963 (DOW CORNING CORP.) * Page 13, paragraph E * --- | 1-8 | |
| Y | GB-A-2 022 091 (AKZO) * Page 4, example 10; page 6, examples 45-93 * --- | 1-8 | |
| Y,P | EP-A-0 077 104 (STAMICARBON) * Page 8 * ----- | 1-8 | |
| | | | TECHNICAL FIELDS SEARCHED (Int. Cl. 3) |
| | | | C 07 C 119/042 |

The present search report has been drawn up for all claims

| Place of search | Date of completion of the search | Examiner |
|---|---|---|
| THE HAGUE | 25-05-1984 | VERHULST W. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document